# EUROPEAN PATENT SPECIFICATION

(11) **EP 3 138 528 B1**
(45) Date of publication and mention of the grant of the patent: **22.01.2020**
(21) Application number: 14890932.8
(22) Date of filing: 01.08.2014
(51) Int. Cl.: A61B 90/00, H02G 3/04, A61G 12/00, H01R 25/14

(54) **MEDICAL PENDANT BOX BODY AND POST USED FOR MEDICAL PENDANT BOX BODY**
MEDIZINISCHER HÄNGENDER KOFFERKÖRPER UND MAST FÜR MEDIZINISCHEN HÄNGENDEN KOFFERKÖRPER
BOÎTIER ALLONGÉ À USAGE MÉDICAL ET COLONNES ASSOCIÉES

(30) Priority: 30.04.2014 CN 201410181261
(43) Date of publication of application: 08.03.2017
(73) Proprietor: Maquet (Suzhou) Co. Ltd., Suzhou, Jiangsu 215024 (CN)
(72) Inventor: HUANG, Jiasheng, Suzhou Jiangsu 215024 (CN); ZHANG, Wei, Suzhou Jiangsu 215024 (CN); LI, Qunhua, Suzhou Jiangsu 215024 (CN); JI, Ming, Suzhou Jiangsu 215024 (CN)
(74) Representative: WSL Patentanwälte Partnerschaft mbB
(86) International application number: PCT/CN2014/083513
(87) International publication number: WO 2015/165160

(56) References cited:
- EP-A1- 2 058 911
- CA-A1- 2 709 726
- CN-A- 103 591 425
- CN-A- 103 919 615
- CN-A- 104 055 576
- CN-U- 201 710 467
- CN-U- 203 815 593
- NL-A- 8 200 247
- US-A1- 2013 307 237

## Description

### Technical field

The present invention relates to the technical field of medical equipment, and more particularly to a medical pendant box body and posts used for the medical pendant box body.

### Background art

Medical pendant is an indispensable medical equipment used in modern hospital operating room, intensive care unit and the like, and various accessories such as handle, laminate and the like are often needed to be fixed (installed) to the medical pendant so as to achieve various functions. The main structure of a medical pendant is a box body. Therefore, in order to achieve the mechanical connection and electrical connection to various medical accessories, various mechanical interfaces and electrical interfaces are needed to be set on the medical pendant box body.

The medical pendant box body of the prior art has the following drawbacks:
(1) Since the structure and/or the appearance of the medical pendant box bodies are different, the modes for the interfaces thereof are also different from each other, and switch connection block is needed to be installed if various interfaces are to be interchanged;
(2) It is needed to complicatedly connect electrical cables with very large time consumption;
(3) The position of a medical accessory is fixed and the height and/or position thereof cannot be freely adjusted.

European patent application EP 2 058 911 A1 is directed to a medical pendant box comprising a coupling rail, which extends along a rectangular, elongated carrier. The coupling rail includes two electrically insulated conductor rails e.g. power supply conductor rail and data transfer conductor rail, which continuously run in the coupling rail. The coupling rail is designed as a hollow rail with an opening along a longitudinal direction. The conductor rails are arranged on an inner side of the hollow rail. A covering device i.e. lip, made of flexible material is provided along the opening, where the covering rail narrows or closes the opening.

### Summary of invention

One of the purposes of the present invention is to overcome the above-mentioned disadvantages of the existing medical pendant box body, there is provided a medical pendant box body which can have standardised interfaces, without complicatedly connecting electrical cables, being easy for disassembly and assembly, and the position of a medical accessory capable of being freely adjusted.

The above purposes of the present invention are achieved by a medical pendant box body, the medical pendant box body comprises at least two panels, at least two posts extending along the longitudinal direction of the medical pendant box body, an upper base plate and a lower base plate, the at least two panels are respectively fixed to the at least two posts, the upper base plate and the lower base plate are respectively fixed to the upper edge and the lower edge of each panel of the at least two panels, at least one post of the at least two posts is provided with an electrical interface, the electrical interface comprises only one inner groove located at the front surface of the post and extending along the longitudinal direction of the post, an insulator accommodated within the inner groove, and a conductor sealed within the insulator.

According to the technical solutions described above, the medical pendant box body of the present invention can have the following advantageous technical effects: a standardised interface can be provided, it is not necessary to connect electrical cables in a complicated manner, disassembly is easy and the position of a medical accessory can be freely adjusted.

Preferably, the inner groove is substantially filled when the insulator is accommodated within the inner groove and the conductor is sealed within the insulator.

According to the technical solutions described above, the medical pendant box body of the present invention can have the following advantageous technical effects: the electrical interface has good reliability and insulation performance, effectively prevents the infiltration of water or other cleaning agents during the cleaning process in a hospital.

Preferably, the number of the insulators within the inner groove is two, and one conductor is sealed within each insulator.

According to the technical solutions described above, the medical pendant box body of the present invention can have the following advantageous technical effects: by means of the appropriate number and settings of the above insulators and conductors, the simple and reliable connection between the plug of the electrical accessory and the conductor inside the post can be achieved.

Preferably, the cross section of the conductors is substantially Y-shaped or Ω-shaped.

According to the technical solutions described above, the medical pendant box body of the present invention can have the following advantageous technical effects: by means of the appropriate shape of the above conductors, the simpler and reliable connection between the plug of the electrical accessory and the conductor inside the post can be achieved.

Preferably, the insulator comprises an insulator front part and an insulator rear part, the conductor is sealed within the insulator rear part, the insulator front part has a substantially flat front surface and a gap extending along the longitudinal direction of the medical pendant box body.

According to the technical solutions described above, the medical pendant box body of the present invention can have the following advantageous technical effects: the gap can be provided for inserting the plug of the electrical accessory, which facilitates the simpler and reliable connection between the plug of the electrical accessory and the conductor inside the post.

Preferably, the width size of the gap and the elasticity of the insulator front part are designed so that the gap can be provided for inserting the plug of an electrical accessory and the insulator front part is substantially sealed before inserting the plug.

According to the technical solutions described above, the medical pendant box body of the present invention can have the following advantageous technical effects: the simpler and reliable connection between the plug of the electrical accessory and the conductor inside the post is achieved, meanwhile effectively preventing the infiltration of water or other cleaning agents during the cleaning process in a hospital.

Preferably, the width size of the inner groove and the elasticity of the insulator are designed to make the insulator produce necessary predeformation so as to produce certain pre-pressure, so that when the plug of an electrical accessory is inserted into the electrical interface to connect to the conductor, the pre-pressure prevents the plug from disengaging from the conductor.

According to the technical solutions described above, the medical pendant box body of the present invention can have the following advantageous technical effects: the reliability of the connection between the plug of the electrical accessory and the conductor is improved, and the disengagement of the plug from the conductor is effectively prevented.

Preferably, the conductor is connected to the control board of the medical pendant via a connector.

According to the technical solutions described above, the medical pendant box body of the present invention can have the following advantageous technical effect: multiple functions such as air brake control, electromagnetic brake control, motor control, direct current supply, video or audio signal supply and the like can be accomplished by means of the control board.

Preferably, at least one post of the at least two posts is also provided with a mechanical interface, the mechanical interface comprising a concave curved portion or a convex curved portion for matching to the convex curved portion or the concave curved portion of a mechanical connecting piece.

According to the technical solutions described above, the medical pendant box body of the present invention can have the following advantageous technical effects: by means of the same post in the medical pendant box body, both the mechanical connection and the electrical connection can be achieved, the number of the components needed is reduced, and the connection reliability is improved; furthermore, a medical accessory is easy to be disassembled and assembled, and the position of the medical accessory can be freely and steplessly adjusted.

Preferably, the mechanical connecting piece is used for installing a medical accessory.

According to the technical solutions described above, the medical pendant box body of the present invention can have the following advantageous technical effects: the medical accessory can be freely adjusted with respect to its height and/or position and can be simply and reliably installed to the medical pendant box body.

The above purposes of the present invention are also achieved by a post used for the medical pendant box body, the post extends along the longitudinal direction of the medical pendant box body, the post is provided with an electrical interface, the electrical interface comprises an inner groove located on the post and extending along the longitudinal direction of the post, an insulator accommodated within the inner groove, and a conductor sealed within the insulator.

According to the technical solutions described above, the post used for the medical pendant box body of the present invention can have the following advantageous technical effects: by means of the innovative design of the post used for the medical pendant box body, the medical pendant box body can possess a standardised interface, without complicatedly connecting electrical cables, being easy for disassembly and assembly, and the position of a medical accessory capable of being freely adjusted.

### Brief description of drawings

Figure 1 is the three-dimensional view for the medical pendant box body of an embodiment of the present invention.
Figure 2 is the three-dimensional view for the medical pendant box body of an embodiment of the present invention, in which the upper base plate is removed for clarity.
Figure 3 is the top view of the medical pendant box body shown in figure 2.
Figure 4 is a partial enlarged view of a portion of the lower part in figure 3, especially showing the electrical interface and the mechanical interface of the post in the medical pendant box body.

### Sign list for drawings:

1. panel;
2. post;
3. upper base plate;
4. lower base plate;
10. medical pendant box body;
21. inner groove;
22. insulator;
23. conductor;
24. connector;
25. concave curved portion;
31. mechanical connecting piece;
35. convex curved portion;
221. insulator front part;
222. insulator rear part;
223. gap.

### Detailed Description

The present invention is further described in connection with drawings and particular embodiments as follows and elaborated in more detail in the following description in order to fully understand the present invention, but it is evident that the present invention can be implemented in many other ways which are different from those described herein; generalization and deduction can be made by a skilled in the art without departing from the connotation of the invention according to practical application, and therefore the protective scope of the present invention should not be limited by the specific content of embodiments of the present invention herein.

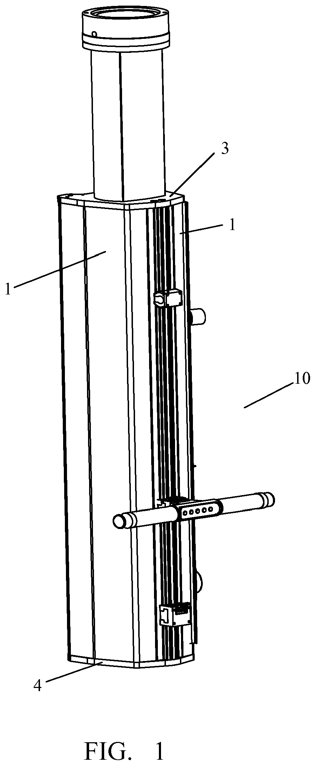

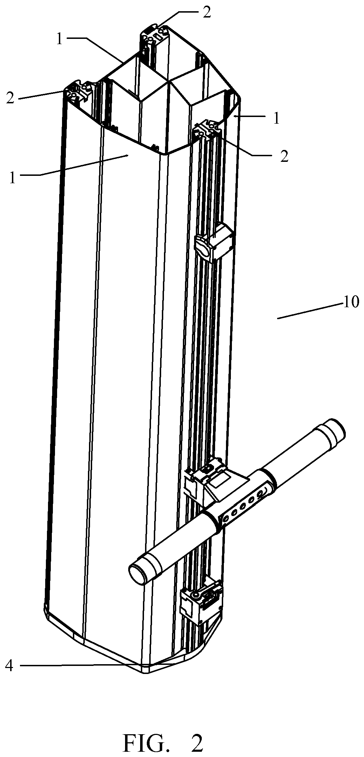

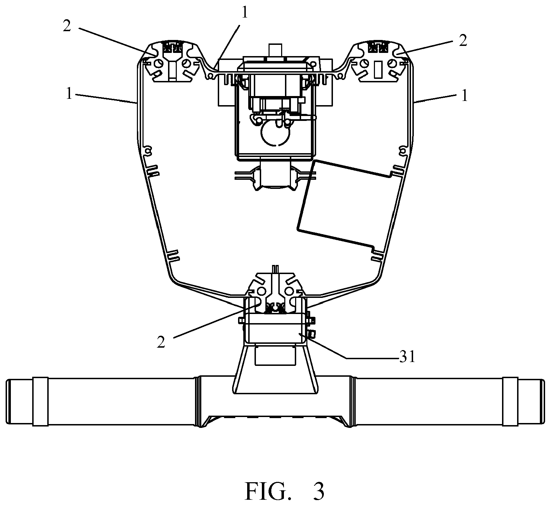

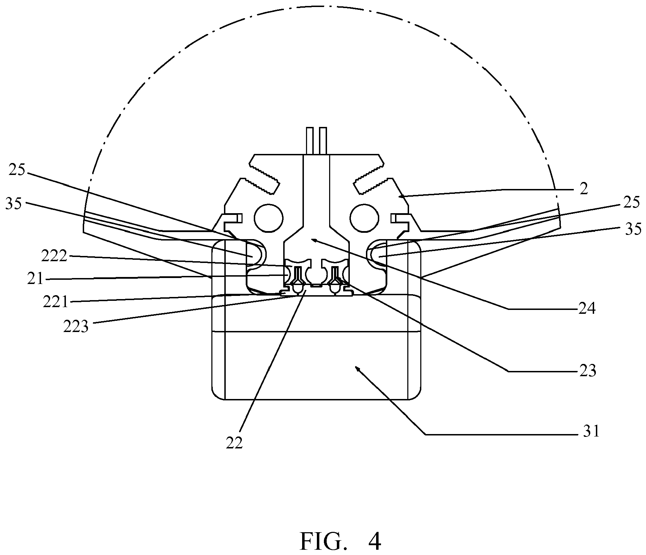

Figure 1 and figure 2 respectively show the three-dimensional views for the medical pendant box body 10 of an embodiment of the present invention, in which the upper base plate is removed from the medical pendant box body 10 in figure 2 for clarity. Figure 3 shows the top view of the medical pendant box body 10 shown in figure 2. Figure 4 shows a partial enlarged view of a portion of the lower part in figure 3, especially showing the electrical interface and the mechanical interface of the post 2 in the medical pendant box body 10.

The medical pendant box body 10 of the present invention comprises at least two panels 1, at least two posts 2 extending along the longitudinal direction of the medical pendant box body, an upper base plate 3 and a lower base plate 4; the at least two panels 1 are respectively fixed to the at least two posts 2, the upper base plate 3 and the lower base plate 4 are fixed to the upper edge and the lower edge of each panel 1 of at least two panels 1, respectively.

Note that, "upper", "lower", "front", "rear", "left", "right" and the like used herein are only exemplary directions defined to facilitate the description of the invention, as shown in figure 3, the direction toward the reader is "upper", the direction away from the reader is "lower", the direction of the bottom side in the paper is "front", the direction of the top side in the paper is "rear", the direction of the left side in the paper is "left", and the direction of the right side in the paper is "right". Of course, those skilled in the art on the basis of the present invention can understood that "upper", "lower", "front", "rear", "left", "right" and other directions can also be defined in other ways, which also fall into the protective scope of the present invention.

As shown in figure 4, at least one post 2 of the at least two posts is provided with an electrical interface, the electrical interface comprises an inner groove 21 located on the post 2 and extending along the longitudinal direction of the post, an insulator 22 accommodated within the inner groove 21, and a conductor 23 sealed within the insulator 22.

In this way, the medical pendant box body of the present invention can possess a standardised electrical interface, without complicatedly connecting electrical cables, being easy for disassembly and assembly, and the position of a medical accessory capable of being freely adjusted.

As shown in figure 4, at least one post 2 of the at least two posts is also provided with a mechanical interface, the mechanical interface comprising a concave curved portion 25 for matching to the convex curved portion 35 of the mechanical connecting piece 31.

In this way, the medical pendant box body of the present invention can possess a standardised mechanical interface, being easy for disassembly and assembly, and the position of a medical accessory capable of being freely adjusted. Furthermore, by means of the same post in the medical pendant box body, both the mechanical connection and the electrical connection can be achieved, the number of the components needed is reduced, and the connection reliability is improved.

Although the examples given above only describe the circumstance with respect to the matching of the concave curved portion of the post to the convex curved portion of the mechanical connecting piece, a person skilled in the art on the basis of the present invention should understand that the form of matching of the convex curved portion of the post to the concave curved portion of the mechanical connecting piece can also be used. Such variation also falls into the protection scope of the present invention.

Preferably, as shown in figures 1 and 2, posts 2 extend along the longitudinal direction of the medical pendant box body 10 and passing through the entire length of the medical pendant box body 10. In this way, by means of a mechanical connecting piece, the location of the medical accessories can be steplessly adjusted along the longitudinal direction of the medical pendant box body.

Preferably, the inner groove 21 extends across the entire length of the post 2. Preferably, as shown in figure 4, the inner groove 21 is located at the front surface of the post 2. In this way, it is easy for the extrusion molding of the post, effectively reducing the processing cost. Preferably, the number of the inner groove 21 on the post 2 is only one. In this way, the simple and reliable electrical connection is realized, meanwhile simplifying the structural design of the post and reducing the processing cost.

Preferably, the insulator 22 extends along the longitudinal direction of the post. Preferably, the insulator 22 also extends across the entire length of the post 2.

Preferably, the conductor 23 extends along the longitudinal direction of the post. Preferably, the conductor 23 also extends across the entire length of the post 2.

In this way, the medical pendant box body of the present invention can freely adjust the position of a medical accessory across the entire length of the post (or the entire length of the medical pendant box body), and easily and quickly realize the electrical connection and/or the mechanical connection.

Preferably, as shown in figure 4, the inner groove 21 is substantially filled when the insulator 22 is accommodated within the inner groove 21 and the conductor 23 is sealed within the insulator 22.

In this way, the electrical interface has good reliability and insulation performance, effectively preventing the infiltration of water or other cleaning agents during the cleaning process in a hospital.

Preferably, as shown in figure 4, the number of the insulators 22 within the inner groove 21 is two, and one conductor 23 is sealed within each insulator 22. Preferably, the insulator 22 comprises an insulator front part 221 and an insulator rear part 222. Preferably, the insulator front parts 221 of the two insulators 22 can be formed integrally so as to effectively prevent the infiltration of water or other cleaning agents during the cleaning process in a hospital.

Preferably, as shown in figure 4, the cross section of the conductor 23 is substantially Y-shaped. Of course, those skilled in the art on the basis of the present invention can understood that the cross section of the conductor 23 can also be substantially Ω-shaped.

In this way, by means of the appropriate settings of the shape and number of the above insulators and conductors, the simple and reliable connection between the plug of the electrical accessory and the conductor inside the post can be achieved.

Preferably, as shown in figure 4, in the case that the insulator 22 comprises an insulator front part 221 and an insulator rear part 222, the conductor 23 is sealed within the insulator rear part 222, the insulator front part 221 has a substantially flat front surface and a gap 223 extending along the longitudinal direction of the medical pendant box body.

Preferably, the insulator 22 (comprising an insulator front part 221 and/or an insulator rear part 222) should possess a certain elasticity. For example, the insulator front part 221 possesses a certain elasticity. Again, for example, the insulator rear part 222 possesses a certain elasticity. Again, for example, both the insulator front part 221 and the insulator rear part 222 possess a certain elasticity.

Preferably, the width size of the gap 223 and the elasticity of the insulator front part 222 are designed so that the gap 223 can be provided for inserting the plug of the electrical accessory and the insulator front part 221 is substantially sealed before inserting the plug.

Preferably, the width size of the inner groove 21 and the elasticity of the insulator 22 are designed to make the insulator 22 (especially insulator rear part 222) produce necessary predeformation so as to produce certain pre-pressure, so that when the plug of an electrical accessory is inserted into the electrical interface to connect to the conductor 23, the pre-pressure prevents the plug from disengaging from the conductor 23.

Preferably, as shown in figure 4, the conductor 23 is connected to the control board of the medical pendant via a connector 24.

Preferably, the mechanical connecting piece 31 can be used for installing various medical accessories, for example, a control handle, an infusion pole, a display arm, a medical guide rail, etc.

The height of the medical accessories can be arbitrarily adjusted after the medical accessories are installed on the interfaces of the posts, and they can be interchanged on different posts.

The conductor 23 provided in post 2 can achieve multiple functions, for example, providing air brake control, electromagnetic brake control, and motor control which are needed for controlling the medical pendant; providing direct current supply, for example, an environment lamp can be directly installed on the conductor; realizing the simultaneous supply of the power and control signals; and supplying video or audio signals, such as the data of a pulsimeter, etc.

The present invention has been exemplarily described above in connection with the figures, although the specific implementations of the present invention are not limited to the above embodiments. Various modifications or variations can be made by a person skilled in the art on the premise of without departing from the technical concept of the present invention, and such modifications or variations of course fall within the protection scope of the present invention.

## Claims

1. A medical pendant box body (10), wherein the medical pendant box body (10) comprises at least two panels (1), at least two posts (2) extending along the longitudinal direction of the medical pendant box body (10), an upper base plate (3) and a lower base plate (4); the at least two panels (1) are respectively fixed to the at least two posts (2), the upper base plate (3) and the lower base plate (4) are respectively fixed to the upper edge and the lower edge of each panel of the at least two panels (1), at least one post of the at least two posts (2) is provided with an electrical interface, **characterised in that** the electrical interface comprises only one inner groove (21) located at the front surface of the post (2) and extending along the longitudinal direction of the post, an insulator (22) accommodated within the inner groove (21), and a conductor (23) sealed within the insulator (22).

2. The medical pendant box body (10) of claim 1, wherein the inner groove (21) is substantially filled when the insulator (22) is accommodated within the inner groove (21) and the conductor (23) is sealed within the insulator (22).

3. The medical pendant box body (10) of claim 1, wherein the number of the insulators (22) within the inner groove (21) is two, and one conductor (23) is sealed within each insulator (22).

4. The medical pendant box body (10) of claim 1, wherein the cross section of the conductor (23) is substantially Y-shaped or Ω-shaped.

5. The medical pendant box body (10) of claim 1, wherein the insulator (22) comprises an insulator front part (221) and an insulator rear part (222), the conductor (23) is sealed within the insulator rear part (222), the insulator front part (221) has a substantially flat front surface and a gap (223) extending along the longitudinal direction of the medical pendant box body (10).

6. The medical pendant box body (10) of claim 5, wherein the width size of the gap (223) and the elasticity of the insulator front part (221) are designed so that the gap (223) can be provided for inserting a plug of an electrical accessory and the insulator front part (221) is substantially sealed before inserting the plug.

7. The medical pendant box body (10) of claim 1, wherein the width size of the inner groove (21) and the elasticity of the insulator (22) are designed to make the insulator (22) produce necessary predeformation so as to produce certain pre-pressure, so that when a plug of an electrical accessory is inserted into the electrical interface to connect to the conductor (23), the pre-pressure prevents the plug from disengaging from the conductor (23).

8. The medical pendant box body (10) of claim 1, wherein the conductor (23) is connected to a control board of the medical pendant via a connector (24).

9. The medical pendant box body (10) of claim 1, wherein at least one post of the at least two posts (2) is also provided with a mechanical interface, the mechanical interface comprising a concave curved portion (25) or a convex curved portion for matching to a convex curved portion (35) or a concave curved portion of a mechanical connecting piece (31).

10. The medical pendant box body (10) of claim 9, wherein the mechanical connecting piece (31) is used for installing a medical accessory.

## Patentansprüche

1. Medizinischer Pendeldosenkörper (10), wobei der medizinische Pendeldosenkörper (10) mindestens zwei Paneele (1), mindestens zwei Pfosten (2), welche sich entlang der Längsrichtung des medizinischen Pendeldosenkörpers (10) erstrecken, eine obere Bodenplatte (3) und eine untere Bodenplatte (4) aufweist, wobei die mindestens zwei Paneele (1) jeweils an den mindestens zwei Pfosten (2) befestigt sind, die obere Bodenplatte (3) und die untere Bodenplatte (4) jeweils an der oberen Kante und der unteren Kante von jedem der mindestens zwei Paneele (1) befestigt sind, wobei mindestens ein Pfosten der mindestens zwei Pfosten (2) eine elektrische Schnittstelle aufweist, **dadurch gekennzeichnet, dass**
die elektrische Schnittstelle nur eine innere Nut (21), welche an der Stirnfläche des Pfostens (2) angeordnet ist und sich entlang der Längsrichtung des Pfostens erstreckt, einen Isolator (22), welcher in der inneren Nut (21) aufgenommen ist und einen Leiter (23), welcher mit dem Isolator (22) abgedichtet ist, aufweist.

2. Medizinischer Pendeldosenkörper (10) nach Anspruch 1, wobei die innere Nut (21) im Wesentlichen gefüllt ist, wenn der Isolator (22) in der inneren Nut (21) aufgenommen ist und der Leiter (23) mit dem Isolator (22) abgedichtet ist.

3. Medizinischer Pendeldosenkörper (10) nach Anspruch 1, wobei die Anzahl an Isolatoren (22) in der inneren Nut (21) zwei beträgt, und in jedem Isolator (22) ein Leiter (23) abgedichtet ist.

4. Medizinischer Pendeldosenkörper (10) nach Anspruch 1, wobei der Querschnitt des Leiters (23) im Wesentlichen Y-förmig oder Ω-förmig ist.

5. Medizinischer Pendeldosenkörper (10) nach Anspruch 1, wobei der Isolator (22) einen vorderen Isolatorteil (221) und einen hinteren Isolatorteil (222) aufweist, wobei der Leiter (23) mit dem hinteren Isolatorteil (222) abgedichtet ist, wobei der vordere Isolatorteil (221) eine im Wesentlichen flache Stirnfläche und einen Spalt (223) aufweist, welcher sich entlang der Längsrichtung des medizinischen Pendeldosenkörpers (10) erstreckt.

6. Medizinischer Pendeldosenkörper (10) nach Anspruch 5, wobei das Breitenmaß des Spalts (223) und die Elastizität des vorderen Isolatorteils (221) so ausgestaltet sind, dass der Spalt (223) zum Einstecken eines Steckers eines elektrischen Zubehörs dienen kann und der vordere Isolatorteil (221) im Wesentlichen vor dem Einstecken des Steckers abgedichtet wird.

7. Medizinischer Pendeldosenkörper (10) nach Anspruch 1, wobei das Breitenmaß der inneren Nut (21) und die Elastizität des Isolators (22) so ausgestaltet sind, dass der Isolator (22) eine notwendige Vorverformung erzeugt, sodass ein gewisser Vordruck erzeugt wird, sodass wenn ein Stecker eines elektrischen Zubehörs in die elektrische Schnittstelle eingesteckt wird, um den Leiter (23) zu verbinden, der Vordruck verhindert, dass der Stecker aus dem Leiter (23) gelöst wird.

8. Medizinischer Pendeldosenkörper (10) nach Anspruch 1, wobei der Leiter (23) über eine Verbindung (24) mit einer Steuertafel des medizinischen Pendels verbunden ist.

9. Medizinischer Pendeldosenkörper (10) nach Anspruch 1, wobei mindestens ein Pfosten der mindestens zwei Pfosten (2) auch mit einer mechanischen Schnittstelle ausgestattet ist, wobei die mechanische Schnittstelle einen konkav gekrümmten Abschnitt (25) oder einen konvex gekrümmten Abschnitt aufweist, welche zu einem konvex gekrümmten Abschnitt (35) oder einem konkav gekrümmten Abschnitt eines mechanischen Verbindungsteils (31) passen.

10. Medizinischer Pendeldosenkörper (10) nach Anspruch 9, wobei das mechanische Verbindungsteil (31) zum Installieren eines medizinischen Zubehörs verwendet wird.

## Revendications

1. Corps de boîtier de suspension médicale (10), le corps de boîtier de suspension médicale (10) comprenant au moins deux panneaux (1), au moins deux montants (2) s'étendant le long de la direction longitudinale du corps de boîtier de suspension médicale (10), une plaque de base supérieure (3) et une plaque de base inférieure (4); les au moins deux panneaux (1) sont respectivement fixés aux au moins deux montants (2), la plaque de base supérieure (3) et la plaque de base inférieure (4) sont respectivement fixées au bord supérieur et au bord inférieur de chaque panneau parmi les au moins deux panneaux (1), au moins un montant parmi les au moins deux montants (2) est muni d'une interface électrique, **caractérisé en ce que**
l'interface électrique comprend une seule gorge interne (21) située au niveau de la surface avant du montant (2) et s'étendant le long de la direction longitudinale du montant, un isolant (22) logé dans la gorge interne (21), et un conducteur (23) scellé à l'intérieur de l'isolant (22).

2. Corps de boîtier de suspension médicale (10) selon la revendication 1, dans lequel la gorge interne (21) est sensiblement remplie lorsque l'isolant (22) est logé à l'intérieur de la gorge interne (21) et le conducteur (23) est scellé à l'intérieur de l'isolant (22).

3. Corps de boîtier de suspension médicale (10) selon la revendication 1, dans lequel le nombre d'isolants (22) dans la gorge interne (21) est de deux, et un conducteur (23) est scellé à l'intérieur de chaque isolant (22).

4. Corps de boîtier de suspension médicale (10) selon la revendication 1, dans lequel la coupe transversale du conducteur (23) est sensiblement en forme de Y ou en forme de Ω.

5. Corps de boîtier de suspension médicale (10) selon la revendication 1, dans lequel l'isolant (22) comprend une partie avant d'isolant (221) et une partie arrière d'isolant (222), le conducteur (23) est scellé à l'intérieur de la partie arrière d'isolant (222), la partie avant d'isolant (221) présente une surface avant sensiblement plate et un espace (223) s'étendant le long de la direction longitudinale du corps de boîtier de suspension médicale (10).

6. Corps de boîtier de suspension médicale (10) selon la revendication 5, dans lequel la taille en largeur de l'espace (223) et l'élasticité de la partie avant d'isolant (221) sont conçues de sorte que l'espace (223) puisse être prévu pour insérer une prise d'un accessoire électrique et la partie avant d'isolant (221) est sensiblement scellée avant l'insertion de la prise.

7. Corps de boîtier de suspension médicale (10) selon la revendication 1, dans lequel la taille en largeur de la gorge interne (21) et l'élasticité de l'isolant (22) sont conçues pour amener l'isolant (22) à produire une pré-déformation nécessaire afin de produire une certaine pré-pression, de sorte que, lorsqu'une prise d'un accessoire électrique est insérée dans l'interface électrique pour se connecter au conducteur (23), la pré-pression empêche la prise de se dégager du conducteur (23).

8. Corps de boîtier de suspension médicale (10) selon la revendication 1, dans lequel le conducteur (23) est connecté à une carte de commande de la suspension médicale par l'intermédiaire d'un connecteur (24).

9. Corps de boîtier de suspension médicale (10) selon la revendication 1, dans lequel au moins un montant parmi les au moins deux montants (2) est également muni d'une interface mécanique, l'interface mécanique comprenant une partie incurvée concave (25) ou une partie incurvée convexe pour s'ajuster à une partie incurvée convexe (35) ou à une partie incurvée concave d'une pièce de connexion mécanique (31).

10. Corps de boîtier de suspension médicale (10) selon la revendication 9, dans lequel la pièce de connexion mécanique (31) est utilisée pour installer un accessoire médical.
